# EUROPEAN PATENT APPLICATION

(11) **EP 1 808 464 A1**
(43) Date of publication of application: **18.07.2007**
(21) Application number: 05790621.6
(22) Date of filing: 07.10.2005
(51) Int. Cl.: C09B 57/00, C07D 231/22, C09B 57/10, G02B 5/22, G09F 9/00

(54) **FILTER FOR ELECTRONIC DISPLAY**

(30) Priority: 07.10.2004 JP 2004295004
(71) Applicant: Kyowa Hakko Chemical Co., Ltd., Tokyo 103-0022 (JP)
(72) Inventor: YAMANO, Junzo c/o Yokkaichi Research Laboratories, Yokkaichi-shi, Mie 510-8520 (JP); UKAI, Katsumi c/o Yokkaichi Research Laboratories, Yokkaichi-shi, Mie 510-8520 (JP)
(74) Representative: Casalonga, Axel
(86) International application number: PCT/JP2005/018607
(87) International publication number: WO 2006/038685

(57) **Abstract**

The present invention provides a filter for electronic display devices comprising a metal complex of squarylium compound represented by General Formula (I): 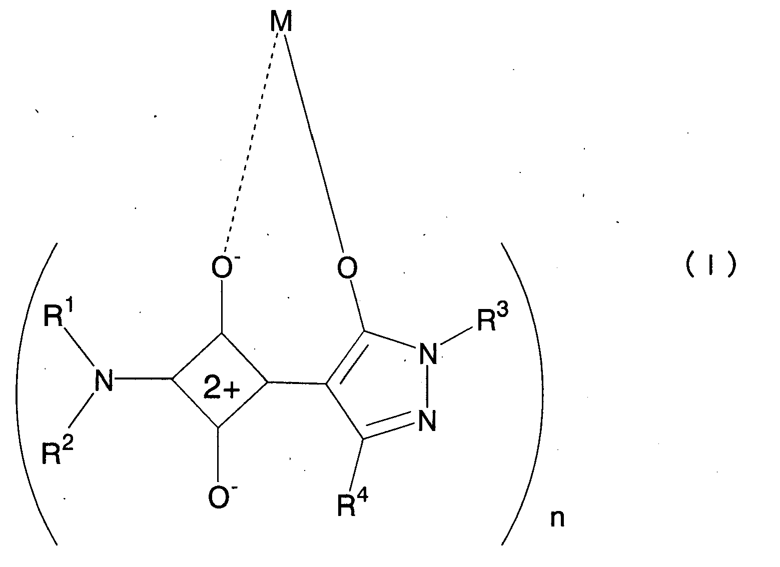 (wherein R¹ and R² may be the same or different and each represents a hydrogen atom, an alkyl group optionally having substituent(s) and the like, R³ and R⁴ may be the same or different and each represents a hydrogen atom, an alkyl group optionally having substituent(s), an aralkyl group optionally having substituent(s) and the like, and M represents a metal atom having coordination function, n represents an integer of 1 to 4).

## Description

### Technical Field

The present invention relates to filters for electronic display devices comprising metal complex of squarylium compounds.

### Background Art

Electronic display devices display color images, ideally, by a combination of three primary colors: red, blue, and green. To display images with clearer colors, it has been devised to equip the devices with filters having color correction functions.

As coloring compounds for filters having color correction functions, squarylium compounds have been used for the purpose of selectively shielding the light having a wavelength of 550 to 600 nm (refer to, for example, Patent Document 1). Also, as colorants for an electronic display device filters that can selectively shield the light having a wavelength of 380 to 450 nm, methine colorants are known (refer to, for example, Patent Document 2), but these colorants were problematic because of their weak light resistance.

In addition, certain metal complexes of squarylium compounds have been used as near-infrared absorbing materials in filters for electronic display devices (refer to, for example, Patent Document 3) and used in optical recording media (refer to, for example, Patent Document 4).
Patent Document 1: Japanese Published Unexamined Patent Application No. 2004-086133
Patent Document 2: Japanese Published Unexamined Patent Application No. 2002-131530
Patent Document 3: Japanese Published Unexamined Patent Application No. 2000-159776
Patent Document 4: WO 2002/50190

### Disclosure of the Invention

### Problem to be Solved by the Invention

An object of the present invention is to provide, for example, filters for electronic display devices which improve color quality of the electronic display devices, etc.

### Means for Solving the Problem

The present invention provides the following [1] to [5]:
[1] A filter for electronic display devices comprising a metal complex of squarylium compound represented by General Formula (I): 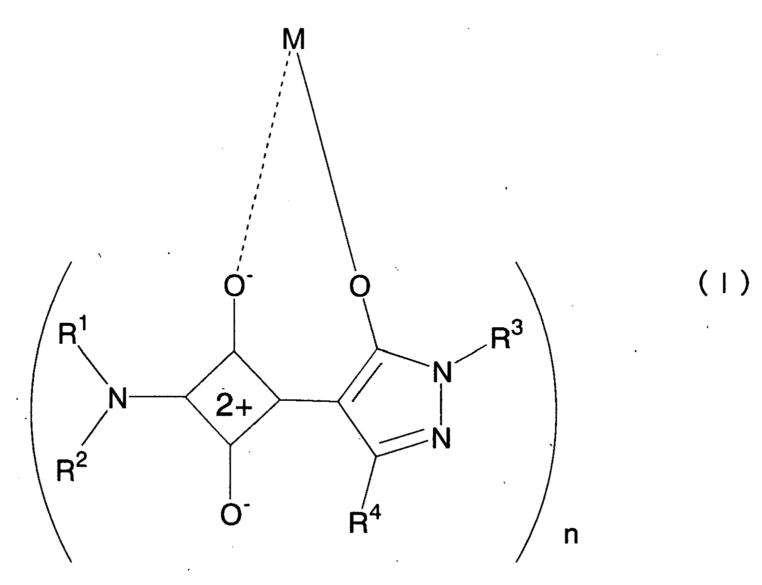 (wherein R¹ and R² may be the same or different and each represents a hydrogen atom, an alkyl group optionally having substituent(s), an alkoxy group optionally having substituent(s), an aralkyl group optionally having substituent(s), an aryl group optionally having substituent(s), or a heterocyclic group optionally having substituent(s), wherein R¹ and R² may be combined together with the adjacent nitrogen atom thereto to form a heterocyclic group optionally having substituent(s), R³ and R⁴ may be the same or different and each represents a hydrogen atom, an alkyl group optionally having substituent(s), an aralkyl group optionally having substituent(s), an aryl group optionally having substituent(s), or a heterocyclic group optionally having substituent(s), M represents a metal atom having coordination function, n represents an integer of 1 to 4).
[2] The filter for electronic display devices according to [1], wherein M represents copper, aluminum, nickel, or zinc.
[3] The filter for electronic display devices comprising a metal complex of squarylium compound according to [1], which has an absorption maximum in a wavelength region of 380 nm to 450 nm.
[4] A metal complex of squarylium compound represented by General Formula (I): 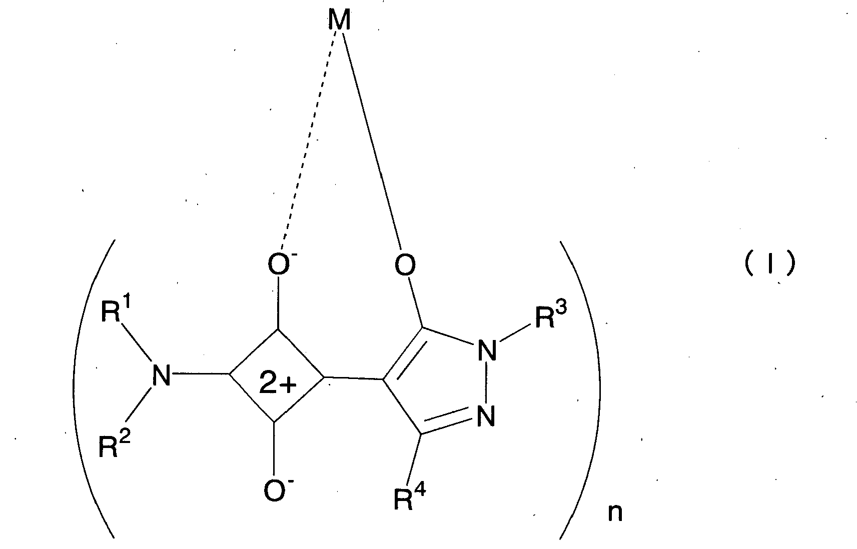 (wherein R¹, R², R³ , R⁴, M and n have the same definitions as described above, respectively).
[5] The metal complex of squarylium compound according to [4], wherein M represents copper, aluminum, nickel, or zinc.

### Effect of the Invention

The present invention provides, for example, filters for electronic display devices which improve the color quality of the electronic display devices, selectively shield a light having a wavelength of preferably 380 nm to 450 nm and provide clearer images, etc.

### Best Mode for Carrying Out the Invention

Hereinafter, the metal complexes of squarylium compounds represented by General Formula (I) are referred to as Compound (I). Compounds with other formula numbers are also expressed in the same manner.

In the definition of each group in the general formulae, examples of the alkyl groups and an alkyl moieties in the alkoxy group include, for example, linear or branched alkyl groups having one to six carbon atoms and cyclic alkyl groups having three to eight carbon atoms, such as a methyl group, an ethyl group, a propyl group, an isopropyl group, a butyl group, an isobutyl group, a sec-butyl group, a tert-butyl group, a pentyl group, an isopentyl group, a 2-methylbutyl group, a tert-pentyl group, a hexyl group, a cyclopropyl group, a cyclobutyl group, a cyclopentyl group, a cyclohexyl group, a cycloheptyl group, and a cyclooctyl group.

Examples of the aralkyl groups include aralkyl groups having seven to fifteen carbon atoms, such as a benzyl group, a phenethyl group, a phenylpropyl group, and a naphtylmethyl group.

Examples of the aryl groups include a phenyl group, a naphthyl group, an anthryl group and the like.

Examples of the heterocyclic rings in the heterocyclic group include heteroaromatic rings (aromatic heterocyclic rings) and alicyclic heterocyclic rings.

Examples of the heteroaromatic rings include 5- or 6-membered monocyclic heteroaromatic rings containing at least one atom selected from nitrogen atoms, oxygen atoms, and sulfur atoms; fused bicyclic or tricyclic heteroaromatic groups containing at least one atom selected from nitrogen atoms, oxygen atoms, and sulfur atoms wherein 3- to 8-membered rings are fused; and the like. More specific examples thereof are a pyridine ring, a pyrazine ring, a pyrimidine ring, a pyridazine ring, a quinoline ring, an isoquinoline ring, a phthalazine ring, a quinazoline ring, a quinoxaline ring, a naphthyridine ring, a cinnoline ring, a pyrrole ring, a pyrazole ring, an imidazole ring, a triazole ring, a tetrazole ring, a thiophene ring, a furan ring, a thiazole ring, an oxazole ring, an indole ring, an isoindole ring, an indazole ring, a benzimidazole ring, a benzotriazole ring, a benzothiazole ring, a benzoxazole ring, a purine ring, a carbazole ring, and the like.

Examples of the alicyclic heterocyclic rings include 5-or 6- membered monocyclic alicyclic heterocyclic rings containing at least one atom selected from nitrogen atoms, oxygen atoms and sulfur atoms; fused bicyclic or tricyclic alicyclic heterocyclic rings containing at least one atom selected from nitrogen atoms, oxygen atoms and sulfur atoms wherein 3- to 8- membered rings are fused; and the like. More specific examples thereof include a pyrrolidine ring, a piperidine ring, a piperazine ring, a morpholine ring, a thiomorpholine ring, a homopiperidine ring, a homopiperazine ring, a tetrahydropyridine ring, a tetrahydroquinoline ring, a tetrahydroisoquinoline ring, a tetrahydrofuran ring, a tetrahydropyran ring, a dihydrobenzofuran ring, a tetrahydrocarbazole ring, and the like.

Examples of the heterocyclic rings in heterocyclic group wherein R¹ and R² are combined together with the adjacent nitrogen atoms thereto, include, for example, 5- or 6- membered monocyclic heterocyclic rings containing at least one nitrogen atom (wherein the monocyclic heterocyclic rings may further contain another nitrogen atom, an oxygen atom, or a sulfur atom); fused bicyclic or tricyclic heterocyclic rings containing at least one nitrogen atom, wherein 3- to 8-membered rings are fused (wherein the fused heterocyclic rings may further contain another nitrogen atom, an oxygen atom, or a sulfur atom); and the like. More specific examples thereof are a pyrrolidine ring, a piperidine ring, a piperazine ring, a morpholine ring, a thiomorpholine ring, a homopiperidine ring, a homopiperazine ring, a tetrahydropyridine ring, a tetrahydroquinoline ring, a tetrahydroisoquinoline ring, a pyrrole ring, an imidazole ring, a pyrazole ring, an indole ring, an indoline ring, an isoindole ring and the like.

Substituents of the alkyl group and the alkoxy group may each have, for example, one to three substituents which may be the same or different. Specific examples of the substituents include a hydroxyl group, a carboxyl group, a cyano group, a halogen atom, an alkoxy group, an alkoxyalkoxy group, an amino group optionally having substituent(s), a nitro group and the like. The alkoxy group has the same definition as described above. The two alkoxy moieties of the alkoxyalkoxy group have the same definitions as the above, respectively. Substituents of the amino group include, for example, 1 or 2 substituent(s) which may be the same or different. Specific examples of the substituents of the amino groups include an alkyl group, an alkoxy group, an aralkyl group, an aryl group and the like. The alkyl group, the alkoxyl group, the aralkyl group and the aryl group have the same definitions as described above, respectively. Examples of the halogen atom include a fluorine atom, a chlorine atom, a bromine atom, and an iodine atom.

Substituents of the aralkyl group, the aryl group, the heterocyclic group, the heterocyclic group formed by combining R¹ and R² together with the adjacent nitrogen atoms are, for example, one to five substituent(s) which may be the same or different. Specific examples of the substituents include an alkyl group, a hydroxyl group, a carboxyl group, a cyano group, a halogen atom, an alkoxy group, an alkoxyalkoxy group, an amino group optionally having substituent(s), a nitro group, and the like. The alkyl group, the halogen atom, the alkoxy group and the alkoxyalkoxy group have the same definitions as described above, respectively. The substituents of the amino group have the same definitions as the above substituents of the amino group.

Examples of the metal atoms having a coordination function include, for example, beryllium, magnesium, aluminum, calcium, titanium, vanadium, chromium, manganese, iron, cobalt, nickel, copper, zinc, molybdenum, iridium and the like. Among them, aluminum, nickel, copper and zinc are preferred.

Compound (I) can be prepared in the following manner.

For example, Compound (I) can be prepared in a similar manner to the Reaction Scheme (1-a) and the Reaction Scheme (1-b). 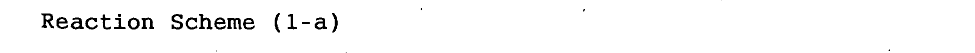 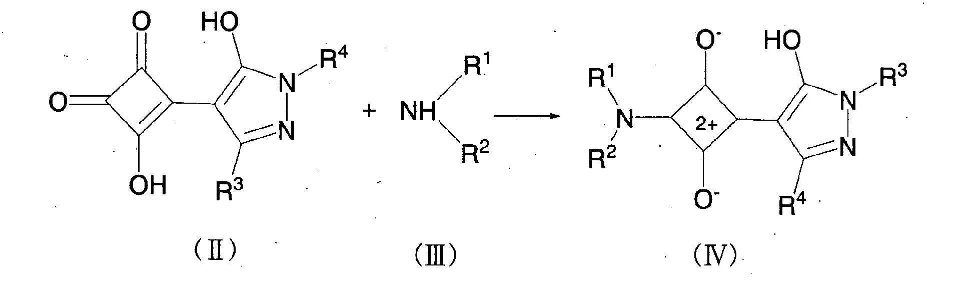 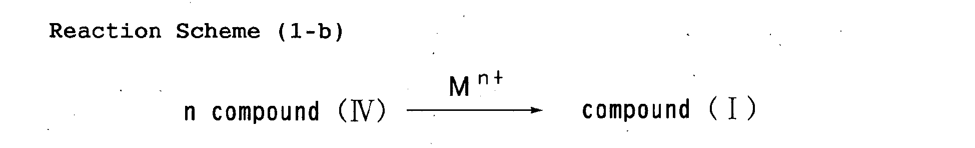 (wherein R¹, R², R³_{;} R⁴, M and n have the same definitions as described above, respectively).

### Reaction Scheme (1-a)

The Compound (II) can be prepared in a similar manner to a known method (e.g., WO 2001/44233 and the like).

The Compound (IV) can be obtained by reacting the Compound (II) with 1- to 5-fold moles of the Compound (III) at a temperature of 80°C to 130°C for one to fifteen hours in a solvent.

Examples of the solvents include an alcohol solvent such as ethanol, propanol, isopropyl alcohol, butanol, or octanol; a mixed solvent of the alcohol solvent with benzene, toluene, or xylene; and the like.

After the reaction, if necessary, the desired compound may be purified by a method generally used in synthetic organic chemistry (such as column chromatography, recrystallization, or washing with a solvent and the like).

### Reaction Scheme (1-b)

The Compound (I) can be prepared in a similar manner to a known method (e.g., WO 2002/050190 and the like).

The Compound (I) may be prepared by reacting a starting material for yielding Mⁿ⁺ and 1 to 4-fold moles of the Compound (IV) in a solvent at a temperature of 25°C to 120°C for one to twenty-four hours, if necessary, in the presence of 1 to 5-fold moles of acetic acid.

Examples of the starting materials for yielding Mⁿ⁺ include, for example, tris(acetylacetonato)aluminum, tris(ethyl acetoacetato)aluminum, aluminium isopropoxide, aluminum sec-butoxide, aluminum ethoxide, aluminium chloride, copper chloride, copper acetate, copper acetylacetonate, nickel acetate, zinc acetylacetonate and the like.

Examples of the solvents include, for example, alcohol solvents such as methanol, ethanol, propanol, isopropyl alcohol, butanol, and octanol; aromatic solvents such as benzene, toluene, and xylene; ester solvents such as ethyl acetate and butyl acetate; halogen-containing solvents such as chloroform and dichloromethane; ether solvents such as tetrahydrofuran and methyl tert-butyl ether; ketone solvents such as acetone and methyl ethyl ketone; and mixtures of these solvents.

After the reaction, if necessary, the desired compound may be purified by a method generally used in synthetic organic chemistry (such as column chromatography, recrystallization, or washing with a solvent).

Preferred examples of the Compound (I) will be illustrated below. The compound numbers in the following table correspond to the Example numbers mentioned below. In the table, "i-Bu" represents an isobutyl group; "Pr" represents a propyl group; and "Ph" represents a phenyl group.

**Table 1**

| Compound | R¹ | R² | R³ | R⁴ | M | n |
|---|---|---|---|---|---|---|
| 1 | -CH₂CH₂OCH₂CH₂- | | Ph | Pr | Al | 3 |
| 2 | -CH₂CH₂OCH₂CH₂- | | Ph | Pr | Cu | 2 |
| 3 | -CH₂CH₂OCH₂CH₂- | | Ph | Pr | Ni | 2 |
| 4 | -CH₂CH₂OCH₂CH₂- | | Ph | Pr | Zn | 2 |
| 5 | i-Bu | i-Bu | Ph | Pr | Cu | 2 |
| 6 | 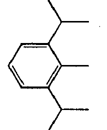 | H | Me | Me | Al | 3 |
| 7 | -CH₂(CH₂)₃CH₂- | | Me | Me | Al | 3 |
| 8 | i-Bu | H | Me | Me | Al | 3 |
| 9 | i-Bu | H | Me | Me | Ni | 2 |
| 10 | i-Bu | H | Me | Me | Cu | 2 |
| 11 | -CH₂(CH₂)₃CH₂- | | Me | CF₃ | Al | 3 |
| 12 | -CH₂(CH₂)₃CH₂- | | Me | CF₃ | Ni | 2 |
| 13 | -CH₂(CH₂)₃CH₂- | | Me | CF₃ | Cu | 2 |
| 14 | i-Bu | H | Me | CF₃ | Ni | 2 |
| 15 | i-Bu | H | Me | CF₃ | Cu | 2 |

The Compound (I) used as filters for electronic display devices of the present invention can be used as colorant of filters for electronic display devices, colorant of two-photon absorption as three-dimensional recording material, sensitizing dye corresponding to short-wavelength laser (for example, blue laser and the like). Among these examples, the filters are suitable as a colorant of filters for electronic display devices because the half maximum full-width in absorption maximum wavelength region is narrow and is excellent in light resistance.

Next, the filters for electronic display devices of the present invention will be illustrated.

Examples of the electronic displays include liquid crystal displays, plasma displays, organic electroluminescence displays, field emission displays and the like.' Among them, plasma displays and the like are preferred.

The Compound (I) used for the filters for electronic display devices of the present invention preferably has an absorption maximum in an absorption region of 380 nm to 450 nm in a chloroform solvent, more preferably, an absorption maximum in an absorption region of 380 nm to 430 nm. The Compound (I) used for the filters for electronic display devices of the present invention also preferably has logarithm of a molar extinction coefficient of 4.5 or more, and more preferably 4.7 or more.

The filters for electronic display devices of the present invention preferably have an absorption maximum in an absorption region of 380 to 450 nm, more preferably, an absorption maximum in an absorption region of 380 nm to 420 nm.

The filters for electronic display devices of the present invention are preferably produced by applying a coating composition containing Compound (I) to an optically transparent substrate, and evaporating an organic solvent. If necessary, another optically transparent substrate may be laminated.

The coating composition may be prepared by dissolving a solution of an organic solvent containing Compound (I) with a binder in the organic solvent.

Examples of the organic solvents include ethers such as dimethoxyethane, methoxyethoxyethane, tetrahydrofuran, and dioxane; ketones such as acetone, methyl ethyl ketone, methyl isobutyl ketone, and cyclohexanone; and aromatic hydrocarbons such as benzene, toluene, xylene, and monochlorobenzene; and the like. These organic solvents are preferably used in an amount 10 to 3000-fold by weight to Compound (I).

Examples of the binders include a polyester resin, a polycarbonate resin, a polyacrylic acid resin, a polystyrene resin, a poly(vinyl chloride) resin, a poly(vinyl acetate) resin and the like. The binder is preferably used in an amount 10- to 500-fold by weight to Compound (I).

The optically transparent substrate is not specifically limited, as long as it comprises an optically transparent resin or glass having low absorption and scattering. Examples of the resin include a polyester resin, a polycarbonate resin, a poly(acrylic acid) resin, a polystyrenic resin, a poly(vinyl chloride) resin, a poly(vinyl acetate) resin and the like.

The coating composition containing the Compound (I) can be applied to the optically transparent substrate according to a known coating procedure, such as bar coating, spraying, roll coating, or dipping (e.g., U.S. Patent No. 2,681,294 and the like).

The Compound (I) has a high solubility in an organic solvent and is suitable for a method of preparing filters for electronic display devices using the above coating composition.

The filters for electronic display devices of the present invention may also be prepared by directly dissolving or dispersing Compound (I) in a resin constituting an optically transparent substrate, forming the solution or dispersion into a film, and, if necessary, laminating the film with other optically transparent substrates at one or both sides thereof.

The film formed from Compound (I) preferably has a half maximum full-width (a width of wavelength region indicating half of the absorbance in an absorption maximum wavelength) of 80 nm or less in an absorption maximum wavelength. The film formed from Compound (I) also preferably has a sufficient transmittance in a region of 445 to 465 nm. For example, in the case of Compound (I) having an absorption maximum in a region of 380 to 430 nm, the resulting film preferably has a transmittance of 65 % or more at 445 to 465 nm, and more preferably 70% or more.

The filters for electronic display devices according to the present invention can selectively shield the light having such a wavelength that reduces the color purity while maintaining the brightness in visible field (can reduce tinting caused by extra blue lights) and are excellent in the color correction functions. Therefore, the filters can provide clear images excellent in colors.

The filters for electronic display devices of the present invention can be used for, for example, cathode-ray tubes, fluorescent display tubes, electroluminescence panels, light emitting diodes, plasma display panels, incandescent lamps, laser displays, liquid crystal displays, electrochromic displays, field emission displays, and the like.

The present invention will be illustrated in further detail with reference to the following Examples and Test Examples.

### EXAMPLE 1: Preparation of Compound 1

Starting material 3-hydroxy-4-[(5-hydroxy-1-phenyl-3-propyl)pyrazol-4-yl]cyclobutene-1,2-dione was synthesized in a similar manner to the method described in WO 2001/44233.

To a mixed solvent of 15 ml of butanol and 15 ml of toluene, 5.00 g of 3-hydroxy-4-[(5-hydroxy-1-phenyl-3-propyl)pyrazol-4-yl]cyclobutene-1,2-dione and 1.90 g of morpholine were added, and the mixture was reacted at 100°C to 120°C for 7.0 hours. Then, the reaction mixture was added with 15 ml of methanol and was cooled to 20°C to 30°C. The precipitated orange solid was collected by filtration (4.53 g).

To 100 mg of the obtained solid, 37.6 mg of tris(ethyl acetoacetato)aluminum, 16.3 mg of acetic acid, and 4 ml of ethyl acetate were added, and the mixture was reacted at 65°C for 4.5 hours. After the reaction mixture was cooled to 20°C, the precipitated yellow solid was collected by filtration to thereby yield Compound 1 (80.0 mg). IR (KBr) cm⁻¹: 2963, 1546, 1478, 1276, 958

### EXAMPLE 2: Preparation of Compound 2

Starting material 3-hydroxy-4-[(5-hydroxy-1-phenyl-3-propyl)pyrazol-4-yl]cyclobutene-1,2-dione was synthesized in a similar manner to the method described in WO 2001/44233.

To a mixed solvent of 15 ml of butanol and 15 ml of toluene, 5.00 g of 3-hydroxy-4-[(5-hydroxy-1-phenyl-3-propyl)pyrazol-4-yl]cyclobutene-1,2-dione and 1.90 g of morpholine were added, and the mixture was reacted at 100°C to 120°C for 7.0 hours. Then, the reaction mixture was added with 15 ml of methanol and was cooled to 20°C to 30°C. The precipitated orange solid was collected by filtration (4.53 g).

To 100 mg of the obtained solid, 24.7 mg of copper acetate, 16.3 mg of acetic acid, and 1 ml of ethyl acetate were added, and the mixture was reacted at 65°C for 5.5 hours. After the reaction mixture was cooled to 20°C, the precipitated brown solid was collected by filtration to thereby yield Compound 2 (70.0 mg). IR (KBr) cm⁻¹: 2960, 1536, 1481, 1276, 959

### EXAMPLE 3: Preparation of Compound 3

Starting material 3-hydroxy-4-[(5-hydroxy-1-phenyl-3-propyl)pyrazol-4-yl]cyclobutene-1,2-dione was synthesized in a similar manner to the method described in WO 2001/44233.

To a mixed solvent of 15 ml of butanol and 15 ml of toluene, 5.00 g of 3-hydroxy-4-[(5-hydroxy-1-phenyl-3-propyl)pyrazol-4-yl]cyclobutene-1,2-dione and 1.90 g of morpholine were added and the mixture was reacted at 100°C to 120°C for 7.0 hours. Then, 15 ml of methanol was added to the reaction mixture and the mixture was cooled to 20°C to 30°C. The precipitated orange solid was collected by filtration (4.53 g).

To 0.50 g of the obtained solid, 0.17 g of nickel acetate tetrahydrate, 0.08 g of acetic acid, 2 ml of ethyl acetate and 2 ml of methanol were added, and the mixture was reacted at 65°C for 5.5 hours. After the reaction mixture was cooled to 20°C, the precipitated yellow-green solid was collected by filtration to thereby yield Compound 3 (0.50 g). IR (KBr) cm⁻¹: 2963, 1575, 1479, 1272, 958

### EXAMPLE 4: Preparation of Compound 4

Starting material 3-hydroxy-4-[(5-hydroxy-1-phenyl-3-propyl)pyrazol-4-yl]cyclobutene-1,2-dione was synthesized in a similar manner to the method described in WO 2001/44233.

To a mixed solvent of 15 ml of butanol and 15 ml of toluene, 5.00 g of 3-hydroxy-4-[(5-hydroxy-1-phenyl-3-propyl)pyrazol-4-yl]cyclobutene-1,2-dione and 1.90 g of morpholine were added and the mixture was reacted at 100°C to 120°C for 7.0 hours. Then, 15 ml of methanol was added to the reaction mixture and the mixture was cooled to 20°C to 30°C. The precipitated orange solid was collected by filtration (4.53 g).

To 0.50 g of the obtained solid, 0.18 g of zinc acetylacetonate, 0.08 g of acetic acid, 2 ml of ethyl acetate and 2 ml of methanol were added, and the mixture was reacted at 65°C for 4.5 hours. After the reaction mixture was cooled to 20°C to 30°C, the precipitated yellow solid was collected by filtration to thereby yield Compound 4 (0.42 g). IR (KBr) cm⁻¹: 2963, 1561, 1476, 1279, 958

### EXAMPLE 5: Preparation of Compound 5

Starting material 3-hydroxy-4-[(5-hydroxy-1-phenyl-3-propyl)pyrazol-4-yl]cyclobutene-1,2-dione was synthesized in a similar manner to the method described in WO 2001/44233.

To a mixed solvent of 1 ml of butanol and 1 ml of toluene, 0.50 g of 3-hydroxy-4-[(5-hydroxy-1-phenyl-3-propyl)pyrazol-4-yl]cyclobutene-1,2-dione and 0.30 g of diisobutylamine were added, and the mixture was reacted at 100°C to 120°C for 10.5 hours. The reaction mixture was added with 1 ml of methanol and was cooled to 0°C. A portion of the reaction mixture was concentrated to thereby yield 1 mg of a solid. The solid (1 mg) was added to the reaction mixture cooled to 0°C, and the precipitated yellow solid was collected by filtration (0.15 g).

To 92.7 mg of the obtained solid, 22.8 mg of copper acetate monohydrate, 13.6 mg of acetic acid, 0.5 ml of ethyl acetate, and 0.5 ml of methanol were added, and the mixture was reacted at 65°C for 5.5 hours. The reaction mixture was concentrated, was added with 5 ml of diisopropyl ether, and was reslurried at 70°C for one hour. After the reaction mixture was cooled to 20°C, the precipitated brown solid was collected by filtration to thereby yield Compound 5 (10.0 mg).
IR (KBr) cm⁻¹: 2960, 1550, 1476, 1282, 1003

### EXAMPLE 6: Preparation of Compound 6

Starting material 3-hydroxy-4-[(1,3-dimethyl-5-hydroxy)pyrazol-4-yl]cyclobutene-1,2-dione was synthesized in a similar manner to the method described in WO 2001/44233.

To a mixed solvent of 10 ml of butanol and 5 ml of toluene, 2.08 g of 3-hydroxy-4-[(1,3-dimethyl-5-hydroxy)pyrazol-4-yl]cyclobutene-1,2-dione and 2.12 g of 2,6-diisopropylaniline were added, and the mixture was reacted at 100°C to 120°C for 7.0 hours. Then, the reaction mixture was added with 5 ml of methanol and was cooled to 20°C to 30°C. The precipitated yellow solid was collected by filtration (1.76 g).

To 0.44 g of the obtained solid, 0.17 g of tris(ethyl acetoacetato)aluminum, 20 mg of acetic acid, and 2 ml of methanol, were added and the mixture was reacted at 65°C for 3 hours. After the reaction mixture was cooled to 25°C, the precipitated yellow solid was collected by filtration to thereby yield Compound 6 (0.42 g). IR (KBr) cm⁻¹: 2964, 1648, 1593, 1491, 1435, 1398, 1363, 1094

### EXAMPLE 7: Preparation of Compound 7

Starting material 3-hydroxy-4-[(1,3-dimethyl-5-hydroxy)pyrazol-4-yl]cyclobutene-1,2-dione was synthesized in a similar manner to the method described in WO 2001/44233.

To a mixed solvent of 4 ml of butanol and 2 ml of toluene, 2.08 g of 3-hydroxy-4-[(1,3-dimethyl-5-hydroxy)pyrazol-4-yl]cyclobutene-1,2-dione and 1.04 g of piperidine were added, and the mixture was reacted at 100°C to 120°C for 8.0 hours. Then, the reaction mixture was added with 5 ml of methanol and was cooled to 20°C to 30°C. The precipitated yellow solid was collected by filtration (1.59 g).

To 0.50 g of the obtained solid, 0.25 g of tris(ethyl acetoacetato)aluminum, 40 mg of acetic acid, and 2 ml of methanol, were added and the mixture was reacted at 65°C for 2.0 hours. After the reaction mixture was cooled to 25°C, the precipitated yellow solid was collected by filtration to thereby yield Compound 7 (0.45 g).
IR (KBr) cm⁻¹: 2942, 1664, 1581, 1548, 1490, 1461, 1448, 1286, 1018, 955

### EXAMPLE 8: Preparation of Compound 8

Starting material 3-hydroxy-4-[(1,3-dimethyl-5-hydroxy)pyrazol-4-yl]cyclobutene-1,2-dione was synthesized in a similar manner to the method described in WO 2001/44233.

To a mixed solvent of 2 ml of butanol and 1 ml of toluene, 1.04 g of 3-hydroxy-4-[(1,3-dimethyl-5-hydroxy)pyrazol-4-yl]cyclobutene-1,2-dione and 0.40 g of isobutylamine were added, and the mixture was reacted at 100°C to 120°C for 5.0 hours. Then, the reaction mixture was cooled to 20°C to 30°C and the precipitated yellow solid was collected by filtration (0.64 g).

To 0.47 g of the obtained solid, 0.25 g of tris(ethyl acetoacetato)aluminum, 40 mg of acetic acid, and 2 ml of methanol, were added and the mixture was reacted at 65°C for 3.0 hours. After the reaction mixture was cooled to 30°C, the precipitated orange solid was collected by filtration to thereby yield Compound 8 (0.45 g). IR (KBr) cm⁻¹: 2960, 1590, 1562, 1489, 1467, 1432, 1031

### EXAMPLE 9: Preparation of Compound 9

Starting material 3-hydroxy-4-[(1,3-dimethyl-5-hydroxy)pyrazol-4-yl]cyclobutene-1,2-dione was synthesized in a similar manner to the method described in WO 2001/44233.

To a mixed solvent of 2 ml of butanol and 1 ml of toluene, 1.04 g of 3-hydroxy-4-[(1,3-dimethyl-5-hydroxy)pyrazol-4-yl]cyclobutene-1,2-dione and 0.40 g of isobutylamine were added, and the mixture was reacted at 100°C to 120°C for 5.0 hours. Then, the reaction mixture was cooled to 20°C to 30°C and the precipitated yellow solid was collected by filtration (0.64 g).

To 0.32 g of the obtained solid, 0.15 g of nickel acetate tetrahydrate, 10.0 mg of acetic acid, and 3 ml of methanol, were added and the mixture was reacted at 65°C for 3.0 hours. After the reaction mixture was cooled to 25°C to 30°C, the precipitated yellow solid was collected by filtration to thereby yield Compound 9 (0.35 g).
IR (KBr) cm⁻¹: 2957, 1580, 1557, 1494, 1463, 1424, 1390

### EXAMPLE 10: Preparation of Compound 10

Starting material 3-hydroxy-4-[(1,3-dimethyl-5-hydroxy)pyrazol-4-yllcyclobutene-1,2-dione was synthesized in a similar manner to the method described in WO 2001/44233.

To a mixed solvent of 2 ml of butanol and 1 ml of toluene, 1.04 g of 3-hydroxy-4-[(1,3-dimethyl-5-hydroxy)pyrazol-4-yl]cyclobutene-1,2-dione and 0.40 g of isobutylamine were added, and the mixture was reacted at 100°C to 120°C for 5.0 hours. Then, the reaction mixture was cooled to 20°C to 30°C and the precipitated yellow solid was collected by filtration (0.64 g).

To 0.32 g of the obtained solid, 0.12 g of copper acetate monohydrate, 10 mg of acetic acid, and 3 ml of methanol, were added and the mixture was reacted at 65°C for 3.0 hours. After the reaction mixture was cooled to 30°C, the precipitated brown solid was collected by filtration to thereby yield Compound 10 (0.32 g). IR (KBr) cm⁻¹: 2960, 1598, 1564, 1551, 1465, 1425, 1389, 1044

### EXAMPLE 11: Preparation of Compound 11

Starting material 3-hydroxy-4-[(5-hydroxy-1-methyl-3-trifluoromethyl)pyrazol-4-yl]cyclobutene-1,2-dione was synthesized in a similar manner to the method described in WO 01/44233.

To a mixed solvent of 2 ml of butanol and 1 ml of toluene, 1.00 g of 3-hydroxy-4-[(5-hydroxy-1-methyl-3-trifluoromethyl)pyrazol-4-yl]cyclobutene-1,2-dione and 0.36 g of piperidine were added, and the mixture was reacted at 100°C to 120°C for 7.0 hours. Then, the reaction mixture was cooled to 20°C to 30°C and the precipitated yellow-green solid was collected by filtration (0.64 g).

To 0.49 g of the obtained solid, 0.21 g of tris(ethyl acetoacetato)aluminum, 30.0 mg of acetic acid, and 3 ml of methanol, were added and the mixture was reacted at 65°C for 3.0 hours. After the reaction mixture was cooled to 30°C, the precipitated yellow solid was collected by filtration to thereby yield Compound 11 (0.48 g).
IR (KBr) cm⁻¹: 2945, 1679, 1587, 1566, 1511, 1487, 1290, 1131, 943

### EXAMPLE 12: Preparation of Compound 12

Starting material 3-hydroxy-4-[(5-hydroxy-1-methyl-3-trifluoromethyl)pyrazol-4-yl]cyclobutene-1,2-dione was synthesized in a similar manner to the method described in

### WO 2001/44233.

To a mixed solvent of 2 ml of butanol and 1 ml of toluene, 1.00 g of 3-hydroxy-4-[(5-hydroxy-1-methyl-3-trifluoromethyl)pyrazol-4-yl]cyclobutene-1,2-dione and 0.36 g of piperidine were added, and the mixture was reacted at 100°C to 120°C for 7.0 hours. Then, the reaction mixture was cooled to 20°C to 30°C and the precipitated yellow-green solid was collected by filtration (0.64 g).

To 0.66 g of the obtained solid, 0.25 g of nickel acetate tetrahydrate, 60.0 mg of acetic acid, 2 ml of ethyl acetate, and 4 ml of methanol were added and the mixture was reacted at 65°C for 5.0 hours. After the reaction mixture was cooled to 30°C, the precipitated brown solid was collected by filtration to thereby yield Compound 12 (0.51 g).
IR (KBr) cm⁻¹: 2939, 1573, 1485, 1448, 1137, 943

### EXAMPLE 13: Preparation of Compound 13

Starting material 3-hydroxy-4-[(5-hydroxy-1-methyl-3-trifluoromethyl)pyrazol-4-yl]cyclobutene-1,2-dione was synthesized in a similar manner to the method described in WO 2001/44233.

To a mixed solvent of 2 ml of butanol and 1 ml of toluene, 1.00 g of 3-hydroxy-4-[(5-hydroxy-1-methyl-3-trifluoromethyl)pyrazol-4-yl]cyclobutene-1,2-dione and 0.36 g of piperidine were added, and the mixture was reacted at 100°C to 120°C for 7.0 hours. Then, the reaction mixture was cooled to 20°C to 30°C and the precipitated yellow-green solid was collected by filtration (0.64 g).

To 0.66 g of the obtained solid, 0.20 g of copper acetate monohydrate, 60.0 mg of acetic acid, 2 ml of ethyl acetate, and 4 ml of methanol were added and the mixture was reacted at 65°C for 5.0 hours. After the reaction mixture was cooled to 30°C, the precipitated brown solid was collected by filtration to thereby yield Compound 13 (0.71 g).
IR (KBr) cm⁻¹: 2946, 1604, 1564, 1486, 1132, 942

### EXAMPLE 14: Preparation of Compound 14

Starting material 3-hydroxy-4-[(5-hydroxy-1-methyl-3-trifluoromethyl)pyrazol-4-yl]cyclobutene-1,2-dione was synthesized in a similar manner to the method described in WO 2001/44233.

To a mixed solvent of 6 ml of butanol and 3 ml of toluene, 3.93 g of 3-hydroxy-4-[(5-hydroxy-1-methyl-3-trifluoromethyl)pyrazol-4-yl]cyclobutene-1,2-dione and 1.32 g of isobutylamine were added, and the mixture was reacted at 100°C to 120°C for 13.0 hours. Then, the reaction mixture was cooled to 20°C to 30°C and the precipitated yellow-green solid was collected by filtration (1.69 g).

To 0.44 g of the obtained solid, 0.17 g of nickel acetate tetrahydrate, 40.0 mg of acetic acid, 1.4 ml of ethyl acetate, and 2.8 ml of methanol were added and the mixture was reacted at 65°C for 5.0 hours. After the reaction mixture was cooled to 30°C, the precipitated yellow solid was collected by filtration to thereby yield Compound 14 (0.44 g). IR (KBr) cm⁻¹: 2961, 1587, 1467, 1430, 11,37, 1014

### EXAMPLE 15: Preparation of Compound 15

Starting material 3-hydroxy-4-[(5-hydroxy-1-methyl-3-trifluoromethyl)pyrazol-4-yl]cyclobutene-1,2-dione was synthesized in a similar manner to the method described in WO 2001/44233.

To a mixed solvent of 6 ml of butanol and 3 ml of toluene, 3.93 g of 3-hydroxy-4-[(5-hydroxy-1-methyl-3-trifluoromethyl)pyrazol-4-yl]cyclobutene-1,2-dione and 1.32 g of isobutylamine were added, and the mixture was reacted at 100°C to 120°C for 13.0 hours. Then, the reaction mixture was cooled to 20°C to 30°C and the precipitated yellow-green solid was collected by filtration (1.69 g).

To 0.44 g of the obtained solid, 0.14 g of copper acetate monohydrate, 40.0 mg of acetic acid, 1.4 ml of ethyl acetate, and 2.8 ml of methanol were added and the mixture was reacted at 65°C for 5.0 hours. After the reaction mixture was cooled to 30°C, the precipitated brown solid was collected by filtration to thereby yield Compound 15 (0.46 g).
IR (KBr) cm⁻¹: 2961, 1561, 1475, 1467, 1430, 1137, 1017

### [TEST EXAMPLE 1]

The absorption maximum wavelength (λmax) and logarithm of a molar extinction coefficient (logε) of Compounds 1 to 15 in a chloroform solvent were measured (800 to 300 nm) usingU-4000 type Spectrophotometer (manufactured by Hitachi Co., Ltd.). The results are shown in Table 2.

**Table 2: Spectroscopic property of metal complexes of squarylium compounds**

| Samples | Spectroscopic property (Chloroform solution) | |
|---|---|---|
| | λmax (nm) | logε |
| Compound 1 | 406.0 | 5.1 |
| Compound 2 | 408.0 | 5.0 |
| Compound 3 | 416.0 | 4.9 |
| Compound 4 | 415.0 | 5.1 |
| Compound 5 | 410.5 | 4.9 |
| Compound 6 | 403.0 | 5.1 |
| Compound 7 | 394.5 | 5.1 |
| Compound 8 | 391.0 | 5.0 |
| Compound 9 | 416.0 | 4.7 |
| Compound 10 | 407.0 | 4.5 |
| Compound 11 | 396.5 | 5.0 |
| Compound 12 | 427.5 | 4.8 |
| Compound 13 | 420.0 | 4.6 |
| Compound 14 | 428.0 | 4.5 |
| Compound 15 | 428.5 | 4.5 |

### [TEST EXAMPLE 2]

Each of a 0.5 percent by weight solution of Compounds 1 to 5 in tetrahydrofuran solution and a 20 percent by weight solution of a polyester resin [VYLON 200 (a product of TOYOBO Co., Ltd.)] in dimethoxyethane solution were mixed at a ratio of 7:2, and the mixture was applied to a glass substrate using a spin coater, and dried to yield a coating film. The absorption maximum wavelength, the half maximum full-width, and the transmittance at 455 nm of the film were measured (800 to 300 nm) using U-4000 type Spectrophotometer (manufactured by Hitachi Co., Ltd.). The results are shown in Table 3.

**Table 3: Absorption maximum wavelengths, half maximum full-widths, and transmittances at 455 nm of metal complexes of squarylium compounds in a film**

| | Absorption maximum wavelength (nm) | Half maximum full-width (nm) | Transmittance at 455 nm (% or more) |
|---|---|---|---|
| Compound 1 | 407.0 | 66.7 | 85 |
| Compound 2 | 411.5 | 79.4 | 85 |
| Compound 3 | 424.0 | 69.8 | 70 |
| Compound 4 | 417.0 | 63.5 | 80 |
| Compound 5 | 414.0 | 69.8 | 85 |

### [TEST EXAMPLE 3]

For evaluating light resistance of coating film obtained from Compound 1 or 2 in Test Example 2, Dewpanel Light Control Weather Meter (manufactured by Suga Test Instruments Co., Ltd., DPWL-5R) was used (irradiation: 15 W/m², chamber temperature: 45 °C, exposure to light: 5 hours). Then, U-4000 type spectrophotometer (manufactured by Hitachi Ltd.) was used to measure transmittance of the coating film before and after the test to obtain colorant residual ratio.

The result of evaluation is shown in Table 4.

**Table 4: Results of light resistance test on films of metal complexes of squarylium compounds**

| | colorant residual ratio (%) |
|---|---|
| Compound 1 | 76.8 |
| Compound 2 | 86.9 |

These results show that the filters for electronic display devices using Compound 1 or 2 are excellent in light resistance, can selectively shield the light having such a wavelength as to reduce the color purity, and can provide clear images.

### Industrial Applicability

The present invention can provide, for example, filters for electric display devices which improve colors of the electric display devices, etc.

## Claims

1. A filter for electronic display devices comprising a metal complex of squarylium compound represented by General Formula (I): 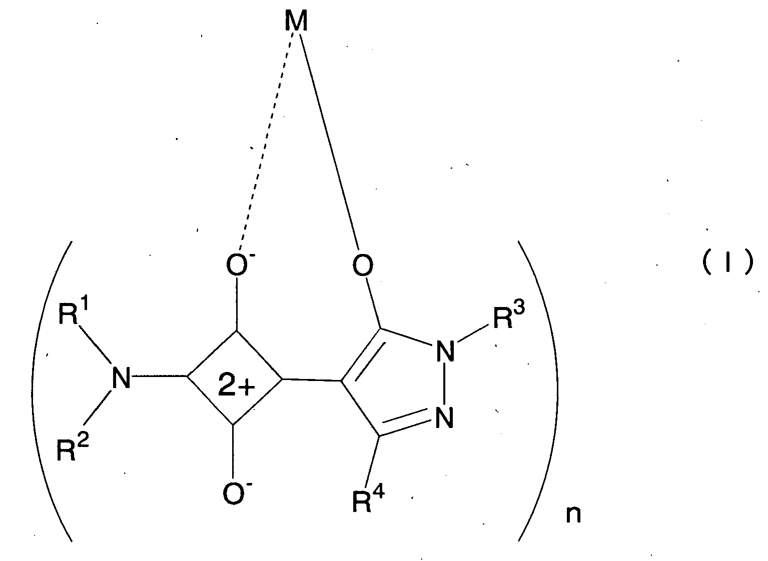 (wherein R¹ and R² may be the same or different and each represents a hydrogen atom, an alkyl group optionally having substituent(s), an alkoxy group optionally having substituent(s), an aralkyl group optionally having substituent(s), an aryl group optionally having substituent(s), or a heterocyclic group optionally having substituent(s), wherein R¹ and R² may be combined together with the adjacent nitrogen atom thereto to form a heterocyclic group optionally having substituent(s), R³ and R⁴ may be the same or different and each represents a hydrogen atom, an alkyl group optionally having substituent(s), an aralkyl group optionally having substituent(s), an aryl group optionally having substituent(s), or a heterocyclic group optionally having substituent(s), M represents a metal atom having coordination function, n represents an integer of 1 to 4).

2. The filter for electronic display devices according to Claim 1, wherein M represents copper, aluminum, nickel, or zinc.

3. The filter for electronic display devices comprising a metal complex of squarylium compound according to Claim 1 or Claim 2, which has an absorption maximum in a wavelength region of 380 nm to 450 nm.

4. A metal complex of squarylium compound represented by General Formula (I): 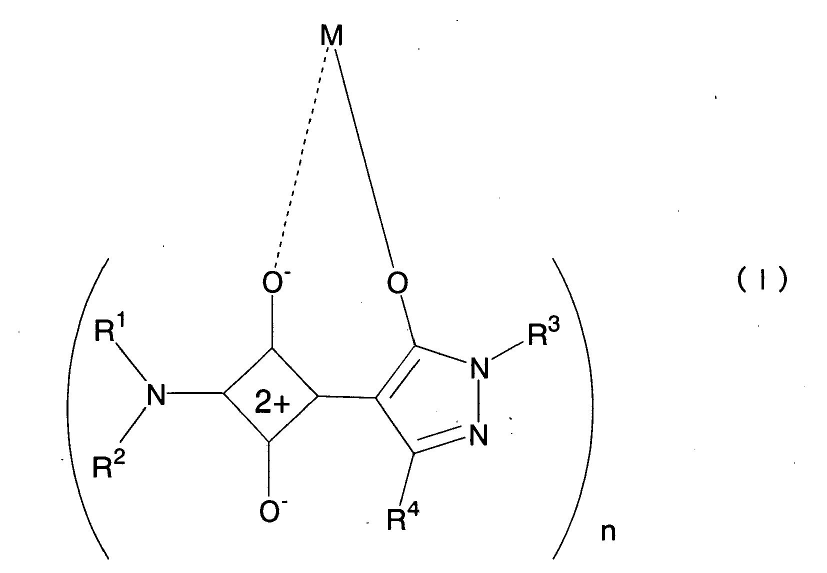 (wherein R¹, R², R³, R⁴, M and n have the same definitions as described above, respectively).

5. The metal complex of squarylium compound according to Claim 4, wherein M represents copper, aluminum, nickel, or zinc.
